(19) **Europäisches Patentamt / European Patent Office / Office européen des brevets**

(11) **EP 2 264 456 A1**

(12) # EUROPÄISCHE PATENTANMELDUNG

(43) Veröffentlichungstag:
**22.12.2010 Patentblatt 2010/51**

(51) Int Cl.:
*G01N 33/50* *(2006.01)*

(21) Anmeldenummer: **10005970.8**

(22) Anmeldetag: **10.06.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **19.06.2009 US 487886**
**23.11.2009 DE 102009054149**

(71) Anmelder:
• **Kief, Thorsten**
  **1430 Rebecq (BE)**

• **Kief, Horst**
  **67069 Ludwigshafen (DE)**

(72) Erfinder:
• **Kief, Thorsten**
  **1430 Rebecq (BE)**
• **Kief, Horst**
  **67069 Ludwigshafen (DE)**

(74) Vertreter: **Wagner, Jutta et al**
  **Patentanwälte**
  **Zellentin & Partner**
  **Rubensstrasse 30**
  **67061 Ludwigshafen (DE)**

(54) **Messung von Schadstoffeinflüssen auf das zelluläre Immunsystem**

(57)    Die Erfindung betrifft ein Verfahren zur Bestimmung der Degranulation der Granulozyten, der Induktion der RNA, der Induktion des PAF (plättchenaktivierenden Faktors), mittels eines Zellcounters, sowie eine Erweiterung des Spektrums des Antigenmaterials durch Umwelttoxine, Tier- oder Pflanzengifte und Tumorzellen. Die Differenzen der Messkanäle (nativ - kontaminiert) werden mathematisch zueinander in Beziehung gesetzt und somit ein hocheffizienter elektronischer Zählmechanismus zur Erfassung des Differentialblutbildes über ein eigenes Computerprogramm genutzt, um die genannten Laborparameter exakt und reproduzierbar zu erfassen.

EP 2 264 456 A1

**Beschreibung**

Die vorliegende Erfindung betrifft ein Messverfahren zur Erfassung von Schadstoffeinflüssen auf das zelluläre Immunsystem. Die Erfindung stellt ein Verfahren zur Bestimmung der Degranulation der Granulozyten, der Induktion der RNA, der Induktion des PAF (plättchenaktivierenden Faktors), mittels eines Zellcounters bereit, sowie eine Erweiterung des Spektrums des Antigenmaterials durch Umwelttoxine, Tier- oder Pflanzengifte und Tumorzellen. Die Differenzen der Messkanäle (nativ - kontaminiert) werden mathematisch zueinander in Beziehung gesetzt und somit ein hocheffizienter elektronischer Zählmechanismus zur Erfassung des Differentialblutbildes über ein eigenes Computerprogramm genutzt, um die genannten Laborparameter exakt und reproduzierbar zu erfassen.

In der Patentanmeldung WO 2008/077638 A1 wird ein Verfahren beschrieben, das sich die einzelnen Messschritte eines Zellcounters zu Nutze macht, um Einflüsse auf das zelluläre Immunsystem des menschlichen Blutes zu bestimmen. Dabei werden die Differenzen, die in den einzelnen Messkanälen bei nativem und kontaminiertem Blut im Bereich der Zellgranula (Neut X), des Zellkerns (Neut Y) und des Volumens der Leukozyten (IMI DC) erfasst und über ein eigenes Computerprogramm ausgewertet. Es werden dabei Messverfahren und die dazu gehörigen mathematischen Formeln beschrieben, die einerseits die Gesamtreaktion der Leukozyten umfassen, andererseits auch durch Hinzuziehung von Monozyten als antigenerkennendem Zellkompartiment die Antigenerkennung besonders gewichtet.

Die nachfolgend beschriebenen mathematischen Berechnungsverfahren erweitern das Gesamtbild der zellulären Immunantwort ganz erheblich, da sich mit diesen Verfahren

- die Zelldegranulation der Granulozyten
- die Induktion der RNA Exkretion aus dem Zellkern und
- die Induktion des PAF, des plättcheninduzierenden Faktors errechnen lässt.

Die Zelldegranulation der Granulozyten ergibt sich auf verblüffend einfache Weise, wenn man die Differenz der Messergebnisse aus Neut X "blank" und Neut X "kontaminiert" durch Neut X "blank" mal Differenz aus IMI DC "blank" und IMI DC "kontaminiert" durch IMI DC "blank" multipliziert, gemäß der nachfolgenden Formel:

$$\text{Leukozyten degranulation} = \frac{\text{Neut X blank} \pm \text{Neut X K}}{\text{Neut X blank}} \times \frac{\text{IMI DC blank} \pm \text{IMI DC K}}{\text{IMI DC blank}}$$

Dabei ist Neut X blank der Messwert des Messkanals des nicht kontaminierten Blutes, Neut X K der Messwert im gleichen Sinne des kontaminierten Blutes, IMI DC der Messwert des gleichnamigen Kanals des Zellcounters unkontaminiert und IMI DC K der Messwert in diesem Sinne kontaminiert.

Die Degranulation der Granulozyten ist eine etablierte Laborbestimmung mit gesicherter Aussage, die ihren Niederschlag bereits in der deutschen Gebührenordnung für Ärzte (GOÄ) gefunden hat. Obige Formel erfasst den morphologischen Zusammenhang:

Eine Minderung der Zellgranula führt zwangsläufig zu einer Schrumpfung der Zelle und beinhaltet somit eine abgeschlossene Degranulation. Andererseits kann eine Zunahme der Granula mit einem Zunehmen des Volumens der Zelle den Aufbau einer Degranulation darstellen oder eine Zunahme der Granula mit einem Schrumpfen des Zellvolumens eine besonders intensive Abwehrphase.

Misst man die Veränderung des Kernvolumens der Granulozyten (Neut Y) im nativen und kontaminierten Zustand und setzt diese Differenz in Beziehung zu Entwicklung der Granulation (Neut X) gemäß der nachfolgenden Formel, lassen sich daraus die Emissionen der RNA aus dem Zellkern ableiten.

$$\text{RNA induktion} = \frac{\text{Neut Y blank} \pm \text{Neut Y K}}{\text{Neut Y blank}} \times \frac{\text{Neut X blank} \pm \text{Neut X K}}{\text{Neut X blank}}$$

Neut Y blank entspricht dabei dem Messwert des Kanals Neut Y des Zellcounters unkontaminiert und Neut Y K dem Messwert im gleichen Sinne kontaminiert.

Die Induktion der RNA lässt sich daraus ableiten, da die RNA als Messengersubstanz aus dem Zellkern stammt und über die spezifischen Organellen im Zytoplasma die Produktion der Granula anregt.

Die Beziehung der Thrombozyten zu allergischen Prozessen neben ihrer Gerinnungsfunktion sowie ihre Position als Endprodukt im Prozess der Induktion des plättchenaktivierenden Faktors sind bekannt.

So hat sich in einigen Labors die Messung der Thrombozytenzahl unter Kontamination mit Allergenen in einigen Fällen als besonders aussagekräftig erwiesen. Bekannt ist auch, dass die Thrombozyten nach bestimmten Reizen ihre Morphologie verändern, d. h. je nach Intensität der Schadeinwirkung an Größe zunehmen.

Beide Maßdifferenzen, die Minderung der Anzahl der Thrombozyten und die Zunahme der Thrombozytengröße setzt die nachfolgende mathematische Formel in Beziehung:

$$\text{PAF induktion} = \frac{\text{Thromb N blank} - \text{Thromb N K}}{\text{Thromb N blank}} \times \frac{\text{Thromb V blank} - \text{Thromb V K}}{\text{Thromb V blank}}$$

Wobei Thromb N blank die Zahl der Thrombozyten unkontaminiert, Thromb N K im gleichen Sinne die Zahl der Thrombozyten kontaminiert darstellt. Thromb V blank repräsentiert das Thrombozytenvolumen unkontaminiert und Thromb V K das Volumen der Thrombozyten kontaminiert.

Da in dieser Formel sowohl die Änderung der Größendifferenz als auch die Änderung der Thrombozytenzahl erfasst werden, ist diese Messtechnik wesentlich sensibler und damit aussagekräftiger als die bisherige alleinige Auszählung der Blutplättchen vor und nach Kontamination.

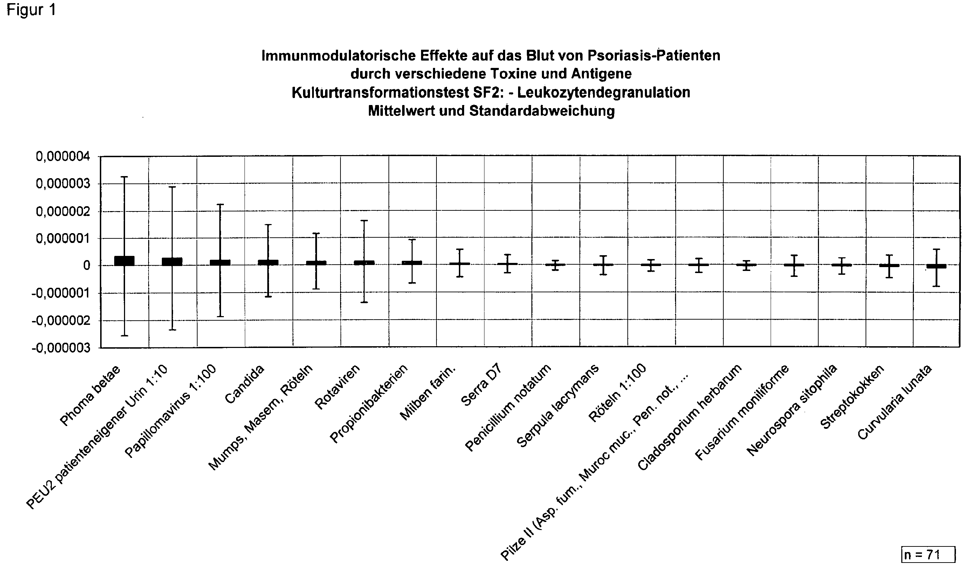

Die Ergebnisse mittels des neuartigen Untersuchungsverfahrens werden anhand der Figure 1 bis 3 demonstriert. Getestet wurden über 50 Arten: Bakterien, Viren und Pilze. Der besseren Übersicht wegen werden nur die relevanten Messergebnisse dargestellt (Mittelwert und Standardabweichung).

Figur 1 zeigt die Ergebnisse der Messungen der Leukozytendegranulation an dem Blut von Psoriasispatienten. Man erkennt eine deutlich positive Reaktion auf Phoma betae, Toxine aus dem eigenen Urin, Papillomaviren 1:100 (Impfstoff) verdünnt, Candida, Masern, Mumps, Röteln Viren (Impfstoff), Rotaviren und Propionibakterien. Eine Negativreaktion zeigen Curvularia lunata, Streptokokken und weitere Sporenträger. Die Reaktion gegen Streptokokken repräsentiert dabei aufgrund des pathologischen Geschehens vor allem Werte bei Psoriasis arthropatica - Patienten.

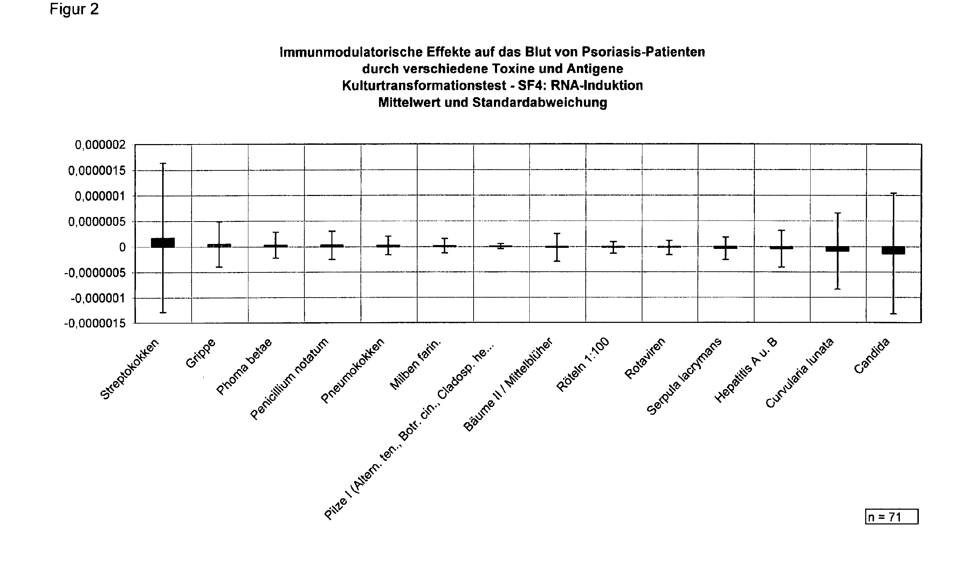

Figur 2 zeigt die Messergebnisse der RNA-Induktion. Es stehen Streptokokken, Grippe, Phoma betae, Penicillium notatum und Pneumokokken im Vordergrund, während sich bei der Negativinduktion Candida, Curvularia lunata, Hepatitis A und B, Serpula lacrymans, Rota- und Rötelviren als relevant erweisen.

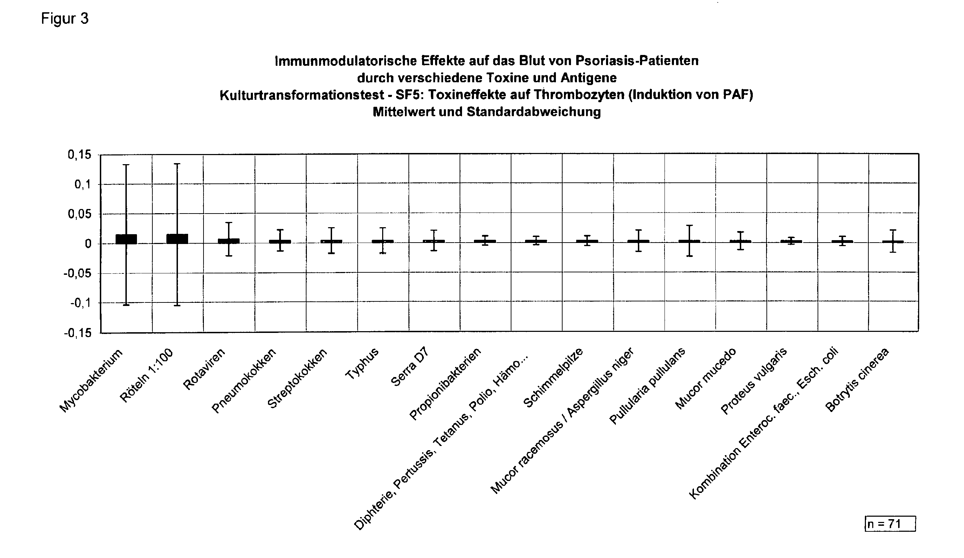

In Figur 3 sind die Ergebisse der PAF-Induktionsmessung dargestellt. Die Thrombozyten von Psoriasispatienten reagieren sehr stark auf Mycobakterium bovis, Röteln, Rotaviren, Pneumokokken, Streptokokken, Typhus, Serratia sowie Propionibakterien, während Pilze nur eine untergeordnete Rolle spielen (daher hier nicht dargestellt). Der Nachweis von Pneumokokken, Streptokokken und Serratia ist bedingt durch den Anteil an Psoriasis arthropatica - Patienten.

Abgesehen von dem derzeitigen Wissensstand, dass es sich bei der Psoriasis um eine Diagnose mit erheblichem Potential zur Vererbung handelt, weisen diese Untersuchungen deutlich auf Cofaktoren als zusätzliche Auslöser dieser multifaktoriellen Erkrankung hin.

Die teilweise Übereinstimmung mit dermatologischen Tests, als auch die Häufigkeit verschiedener Streptokokkenarten bei Psoriasis nach diesen Untersuchungen sprechen für die Validität dieses Testverfahrens.

Der Nachweis von antimikrobiellen Peptiden gegen

- Escherichia Coli
- Staphylokokkus aureus
- Pseudomonas aeruginosa
- Candida albicans
- Acinetobacter Baumannii
- Propionibacterium acnes
- Streptokokkus pyogenes
- Streptokokkus Pneumoniae
- Enterococcus faecalis

in Psoriasisherden stimmt überraschend häufig mit den eigenen Ergebnissen überein und bestätigt somit den Wert dieses Messverfahrens. Unterschiede ergeben sich im wesentlichen nur durch das untersuchte Erregerspektrum.

Autologe Blutkulturen, die mit diesen Keimen nach individueller Austestung gezüchtet werden, bewirken in 70 - 80 % der Fälle Remissionen, auch Vollremissionen, die zu einer weit längeren Erscheinungsfreiheit bei der Psoriasis führen, als dies mit bislang geübten konventionellen Verfahren möglich war.

Das Kontaminationsmaterial kann dabei nicht nur Bakterien, Viren und Pilze umfassen, sondern auch umwelttoxische Substanzen wie z. B. Insektizide, Herbizide, Konservierungsmittel für Nahrung, Holzschutzmittel, Chemikalien, usw. oder Tier- und Pflanzengifte.

Auch denaturierte Tumorzellen (z. B. durch Kälte) können als Antigenmaterial dienen. Besonders die Leukozytendegranulation spricht auf dieses Material an und kann damit als Maß für eine eventuelle Effektivität im Rahmen einer Immuntherapie dienen.

**Patentansprüche**

1. Messverfahren zur Bestimmung der Immunantwort des zellulären Immunsystems, **dadurch gekennzeichnet, dass** die Degranulation der Granulozyten aus den Differenzen von Granulation und Zellvolumen entsprechend den gleichnamigen Messwerten eines Zellcounters Neut X blank der Wert des Messkanals des nicht kontaminierten Blutes, Neut X K der Wert des kontaminierten Blutes, IMI DC der Wert unkontaminiert und IMI DC K der Wert kontaminiert gemäß der Formel

$$\text{Leukozyten degranulation} = \frac{\text{Neut X blank} \pm \text{Neut X K}}{\text{Neut X blank}} \times \frac{\text{IMI DC blank} \pm \text{IMI DC K}}{\text{IMI DC blank}}$$

errechnet wird.

2. Verfahren nach Anspruch 1, **dadurch gekennzeichnet, dass** die Induktion der nukleären RNA-Exkretion durch einen kontaminierenden Schadstoff aus den Differenzen des Kernvolumens und der Zellgranula gemäß nachfolgender Formel

$$\text{RNA induktion} = \frac{\text{Neut Y blank} \pm \text{Neut Y K}}{\text{Neut Y blank}} \times \frac{\text{Neut X blank} \pm \text{Neut X K}}{\text{Neut X blank}}$$

aus den gleichnamigen Messwerten eines Zellcounters Neut Y blank der Wert des Messkanals des nicht kontaminierten Blutes,
Neut Y K der Wert des kontaminierten Blutes und Neut X blank der Wert des Messkanals des nicht kontaminierten Blutes,
Neut X K der Wert des kontaminierten Blutes
bestimmt wird.

3. Verfahren nach Anspruch 1 und 2, **dadurch gekennzeichnet, dass** der toxische Effekt eines Allergens oder einer sonstigen Noxe chemischer oder infektiöser Natur auf die Thrombozyten, insbesondere die Induktion des PAF (plättchenaktivierenden Faktors) durch Messen der Differenzen des Volumens und der Anzahl der Thrombozyten (nativ - kontaminiert) gemäß nachfolgender Formel

$$\text{PAF induktion} = \frac{\text{Thromb N blank} - \text{Thromb N K}}{\text{Thromb N blank}} \times \frac{\text{Thromb V blank} - \text{Thromb V K}}{\text{Thromb V blank}}$$

bestimmt wird.

4. Verfahren nach einem der Ansprüche 1 bis 3, **dadurch gekennzeichnet, dass** es zur Virulenzbestimmung von klassischem Antigenmaterial wie Viren, Bakterien, Pilze und Allergenen dient.

5. Verfahren nach einem der Ansprüche 1 bis 3, **dadurch gekennzeichnet, dass** es zur Virulenzbestimmung von Tier- und Pflanzengiften, umwelttoxischen Schadstoffen dient.

6. Verfahren nach einem der Ansprüche 1 bis 3, **dadurch gekennzeichnet, dass** es zur Bestimmung der zellulären Immunantwort auf Tumormaterial dient.

Figur 1

**Immunmodulatorische Effekte auf das Blut von Psoriasis-Patienten
durch verschiedene Toxine und Antigene
Kulturtransformationstest SF2: - Leukozytendegranulation
Mittelwert und Standardabweichung**

EP 2 264 456 A1

n = 71

Figur 2

Figur 3

Immunmodulatorische Effekte auf das Blut von Psoriasis-Patienten
durch verschiedene Toxine und Antigene
Kulturtransformationstest - SF5: Toxineffekte auf Thrombozyten (Induktion von PAF)
Mittelwert und Standardabweichung

EP 2 264 456 A1

EP 2 264 456 A1

Europäisches Patentamt
European Patent Office
Office européen des brevets

**EUROPÄISCHER RECHERCHENBERICHT**

Nummer der Anmeldung

EP 10 00 5970

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | LINSSEN J ET AL: "Automation and validation of a rapid method to assess neutrophil and monocyte activation by routine fluorescence flow cytometry in vitro" CYTOMETRY, Bd. 74B, Nr. 5, September 2008 (2008-09), Seiten 295-309, XP002599978 * Seite 302, linke Spalte, Absatz 5 - Seite 303, rechte Spalte, Absatz 5; Abbildungen 2,5 * ----- | 1-6 | INV. G01N33/50 |
| X,D | WO 2008/077638 A1 (KIEF HORST [DE]) 3. Juli 2008 (2008-07-03) * Absatz [0035]; Abbildungen 3,6 * ----- | 1-6 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| München | 15. September 2010 | Fleitmann, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

# EP 2 264 456 A1

**ANHANG ZUM EUROPÄISCHEN RECHERCHENBERICHT
ÜBER DIE EUROPÄISCHE PATENTANMELDUNG NR.**

EP 10 00 5970

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-09-2010

| Im Recherchenbericht angeführtes Patentdokument | Datum der Veröffentlichung | Mitglied(er) der Patentfamilie | Datum der Veröffentlichung |
|---|---|---|---|
| WO 2008077638 A1 | 03-07-2008 | EP 2120975 A1<br>US 2010003270 A1 | 25-11-2009<br>07-01-2010 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- WO 2008077638 A1